(19) Europäisches Patentamt
European Patent Office
Office européen des brevets

(11) Publication number: 0 005 219
B1

(12) # EUROPEAN PATENT SPECIFICATION

(45) Date of publication of patent specification: 22.07.81

(51) Int. Cl.³: C 07 D 233/64, A 61 K 31/415

(21) Application number: 79101196.8

(22) Date of filing: 20.04.79

(54) **Arylimidazoles, their synthesis and pharmaceutical or veterinary preparations containing them.**

(30) Priority: 24.04.78 GB 1612078
02.10.78 DK 4357/78

(43) Date of publication of application:
14.11.79 Bulletin 79/23

(45) Publication of the grant of the European patent:
22.07.81 Bulletin 81/29

(84) Designated Contracting States:
BE CH DE FR GB IT LU NL SE

(56) References cited:
DE - A - 1 445 628
DE - A - 2 533 211
GB - A - 997 396
GB - A - 1 047 569

JOURNAL OF ORGANIC CHEMISTRY, vol. 36, no. 16, August 13, 1971, Washington (USA)
R. L. COHEN: "Substituent Effects on the reactivity of triarylimidazolyl free radical towards tris-(2-methyl-4-diethyl-aminophenyl)methane", pages 2280—4

(73) Proprietor: A/S Dumex (Dumex Ltd.)
37, Prags Boulevard Post Box No. 1736
DK-2300 Copenhagen S. (DK)

(72) Inventor: Dyrsting, Hjarne
Geelskovvej 17
DK-2830 Virum (DK)
Inventor: Jorgensen, Dan
Gongesletten 27
DK-2950 Vedbaek (DK)

(74) Representative: Hauck, Hans, Dipl.-Ing. et al,
Patentanwälte Dipl.-Ing.H.Hauck, Dipl.-Phys.W.Schmitz, Dipl.-Ing.E.Graalfs, Dipl.-Ing.W.Wehnert, Dipl.-Phys.W.Carstens Dr.-Ing.W.Döring Neuer Wall 41
D-2000 Hamburg 36 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 005 219 B1

Courier Press, Leamington Spa, England.

Arylimidazoles, their synthesis and pharmaceutical or veterinary preparations containing them.

The present invention relates to novel imidazole derivatives and to processes for their preparation. The novel compounds possess interesting physiological properties.

Certain substituted imidazoles have been described in the literature and these have been prepared in a number of different ways and for various purposes.

In Davidson, Weiss and Jelling: J. Org. Chem. *2*, 319 (1937); Brederech and Theilig: Chem. Ber. *86*, 88 (1953); Brederech, Gompper and Hayer: Chem. Ber. *92*, 338 (1959); White and Sonnenberg: J. Org. Chem. *29*, 1926 (1964); Ogata, Kawasaki and Sugiura: J. Org. Chem. *34*, 3981 (1969); H. Lettau: Z. Chem. *11*, 10 (1971) and Wegner and Schunack: Arch. Pharmaz. *307*, 492 (1974) various imidazoles are prepared without any indication as to their utility.

Radziszewski: Chem. Ber. *10*, 70 (1877), Cook and Jones: J. Chem. Soc. 278 (1941) and others have found that a number of arylimidazoles exhibit chemiluminescence under certain conditions. This property can be utilized in certain copying techniques (e.g. xerography) and forms the basis of German Patent No. 1,106,599, Belgian Patent No. 585,555 and French Patent No. 1,351,818.

Certain 2,4,5-triarylimidazoles have been tested as anti-fertility agents by Bhaduri and Khanna: Indian J Chem. *4*, 419 (1966).

Certain 2-alkyl-4,5-diarylimidazoles are described in German Offenlegungsschrift No. 2,064,520 as possessing analgesic, antiinflammatory and antipyretic activity. One of the preferred compounds of this series, 2-isopropyl-4,5-bis(*p*-methoxyphenyl) imidazole, has been included in the pharmacological tests described hereinafter, from which it can be seen that this preferred compound possesses a much weaker analgesic activity than the compounds of the present invention.

Certain 2,4,5-trisubstituted imidazoles containing a trifluoromethyl group in one of these positions and in some cases also substituted in the 1-position have been described in German Offenlegungsschrift No. 2,155,558 (cf. Lombardino and Wieseman: J. Med. Chem. *17*, 1182 (1974)). The preferred compound of this series, 2-trifiuoromethyl-4,5-bis(*p*-methoxyphenyl) imidazole, has been included in the pharmacological data hereinafter described from which it will be seen that tests indicate this preferred compound to possess a weaker analgesic activity, but a higher toxicity than the compounds of the present invention.

Our British Patent No. 1,469,532 describes and claims certain imidazoles substituted in the 1-position by an acetic acid or propionic acid group or ester thereof. Our investigations on animal models conventionally employed in pharmacology using such compounds show that in general, arylimidazoles substituted in the 1-position by a propionic acid group are more active than the corresponding imidazole unsubstituted in the 1-position.

The present invention is based on the discovery of certain 2-(fluoro-substituted phenyl)-4,5-bis(*p*-methoxy phenyl)-imidazoles unsubstituted in the 1-position which possess substantially improved properties over the compounds disclosed in German Offenlegungsschrift Nos. 2,064,520 and 2,155,558 and more particularly show improved activity over 1-(2-carboxyethyl)-2-(*p*-fluorophenyl)-4,5-bis(*p*-methoxyphenyl) imidazole, a corresponding imidazole substituted in the 1-position by a propionic acid group.

Thus according to the present invention there are provided compounds of the formula:

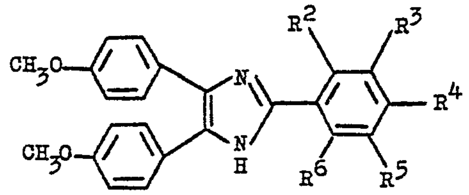

I

wherein one or two of the substituents $R^2$, $R^3$, $R^4$, $R^5$ and $R^6$ represent a fluorine atom while the remaining substituents are hydrogen atoms.

Examples of compounds according to the present invention are:

2-(2-Fluorphenyl)-4,5-bis(p-methoxyphenyl)imidazole
2-(3-Fluorphenyl)-4,5-bis(p-methoxyphenyl)imidazole
2-(4-Fluorphenyl)-4,5-bis(p-methoxyphenyl)imidazole
2-(2,3-Difluorphenyl)- 4,5-bis(p-methoxyphenyl)imidazole
2-(2,4-Difluorphenyl)-4,5-bis(p-methoxyphenyl)imidazole
2-(2,5-Difluorphenyl)-4,5-bis(p-methoxyphenyl)imidazole
2-(2,6-Difluorphenyl)-4,5-bis(p-methoxyphenyl)imidazole
2-(3,4-Difluorphenyl)-4,5-bis(p-methoxyphenyl)imidazole
2-(3,5-Difluorphenyl)-4,5-bis(p-methoxyphenyl)imidazole

Tests which we have conducted show the compounds of the invention are potent prostaglandin synthesis inhibitors both *in vitro* and *in vivo* and possess interesting pharmacological properties. In particular the compounds of the invention exhibit good analgesic, antiinflammatory and antipyretic activity whist possessing low ulcerogenicity and toxicity.

The potency of the compounds of formula I is of the same order of magnitude.

The compounds of the present invention were submitted to a pharmacological screening programme comprising the following tests:

*Tests for analgesic activity*

1. Writhing test in mice. (SPF females of the strain NMRI/BOM, weighing 20—25 g.). The test substance was administered by gavage ½ hour prior to an intraperitoneal injection of acetic acid. The number of writhing movements in 20 minutes was counted.

2. Randall-Selitto test in rats. (SPF males of the strain Sprague-Dawley, weighing 90—100 g). The test was performed on the whole as described by Randall and Selitto: Arch. Int. Pharmacodyn. Ther. *111*, 409 (1957). An analgesia-meter of the make Ugo Basile was used and the test substance was administered by gavage 2 hours after injection of a suspension of brewer's yeast into the rat paw.

*Test for antiinflammatory activity in rats*

SPF males of the strain Sprague-Dawley, weighing ~150 g were used. The test was performed according to Winter *et al.:* Proc. Soc. Exp. Biol. Med. *111*, 544 (1962). The test substance was given by gavage 1 hour prior to injection of a carrageenin-suspension into the rat paw.

*Test for acute toxicity ($LD_{50}$) in mice*

The test substance was administered by gavage. Observation period: 168 hours.

*Ulcerogenic activity in rats*

SPF males of the strain Sprague-Dawley weighing 180—250 g were used. 5 hours after administration by gavage of the test substance the rats were killed by an overdose of ether. The stomachs were removed and opened along the greater curvature. The number of ulcers was assessed and compared to control rats.

*Prostaglandin-synthetase inhibition test*

Performed on the whole according to Vane: Nature New Biol. *231, 232 (1972).*

*Platelet aggregation inhibition test*

The ability of the test substance to inhibit arachidonic acid induced aggregation of human platelet rich plasma was tested in a HU aggregometer (from H. Upchurch & Co. Ltd.).

Table 1 comprises the results of the pharmacological screening of the following fifteen compounds:

| Compound No. | |
|---|---|
| 1. | 1-(2-Carboxyethyl)-2-(p-fluorphenyl)-4,5-bis(p-methoxyphenyl)imidazole. |
| 2. | 2-(o-Fluorphenyl)-4,5-bis(p-methoxyphenyl)imidazole. |
| 3. | 2-(m-Fluorphenyl)-4,5-bis(p-methoxyphenyl)imidazole. |
| 4. | 2-(p-Fluorphenyl)-4,5-bis(p-methoxyphenyl)imidazole. |
| 5. | 2-(2,3-Difluorphenyl)-4,5-bis(p-methoxyphenyl)imidazole. |
| 6. | 2-(2,4-Difluorphenyl)-4,5-bis(p-methoxyphenyl)imidazole. |
| 7. | 2-(2,5-Difluorphenyl)-4,5-bis(p-methoxyphenyl)imidazole. |
| 8. | 2-(2,6-Difluorphenyl)-4,5-bis(p-methoxyphenyl)imidazole. |
| 9. | 2-(3,4-Difluorphenyl)-4,5-bis(p-methoxyphenyl)imidazole. |
| 10. | 2-(3,5-Difluorphenyl)-4,5-bis(p-methoxyphenyl)imidazole. |
| 11. | 2-Trifluormethyl-4,5-bis(p-methoxyphenyl)imidazole. |
| 12. | 2-Isopropyl-4,5-bis(p-methoxyphenyl)imidazole. |
| 13. | Indomethacin. |
| 14. | Naproxen. |
| 15. | Acetyl salicylic acid. |

TABLE 1

The doses stated for $ED_{50}$ and $LD_{50}$ are mg/kg

| Compound No. | Analgesic $ED_{50}$ | | Antiinflammatory test $ED_{50}$ | Acute toxicity $LD_{50}$ | Ulcerogenic activity $ED_{50}$ | Inhibition of PG-synthetase $IC_{50}$ µg/10 µg AA⁺ | Inhibition of platelet aggregation $IC_{100}$ µg190 µg AA⁺ |
|---|---|---|---|---|---|---|---|
| | Writhing test | Analgesia-meter | | | | | |
| 1 | >200 | | | >2000 | | | |
| 2 | 15 | 0,3 | 130 | >2000 | 590 | | |
| 3 | 3,2 | 0,25 | 38 | >2000 | 870 | | |
| 4 | 5 | 0,3 | 14 | 1900 | 360 | 0,18 | 0,048 |
| 5 | 4,6 | 0,55 | 120 | >2000 | 800 | 0,21 | 0,024 |
| 6 | 4 | 0,8 | 47 | >2000 | >300 | 0,27 | 0,12 |
| 7 | 16 | 0,55 | 110 | >2000 | 1000 | 1,71 | 0,024 |
| 8 | 6,8 | 0,55 | 300 | >2000 | >300 | 0,13 | 0,012 |
| 9 | 1,9 | 0,062 | 15 | >2000 | | | 0,006 |
| 10 | 10 | 0,45 | 150 | | 780 | | |
| 11 | 75 | | 25 | >2000 | 450 | | 0,06 |
| 12 | 200 | | | >2000 | | | |
| 13 | 8 | 0,36 | 28 | 21 | 9,7 | 0,65 | 1,19 |
| 14 | 33 | 1,6 | 10 | 1340 | 19,5 | 0,49 | 4,76 |
| 15 | 100 | | 350 | 1700 | 340 | 7,61 | 2,38 |

AA⁺ = Arachidonic acid

The compounds of the present invention may be prepared by the following processes:

a) the reaction of anisil and ammonium acetate with a compound of the formula:—

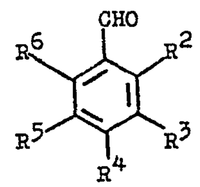

II

where one or two of the substituents $R^2$, $R^3$, $R^4$, $R^5$ and $R^6$ represent a fluorine atom, while the remaining substituents represent hydrogen atoms, whereby a compound of formula I as hereinbefore defined is obtained.

The reaction may, for example, be effected in a manner analogous to the method of White and Sonnenberg: J. Org. Chem. *29*, 1926 (1964). Thus anisil, ammonium acetate and the appropriate aldehyde are reacted together conveniently in the presence of a protic solvent, preferably acetic acid.

It is convenient to use the anisil and aldehyde in equimolar quantities in which case it is advantageous to use about five times the equimolar amount of ammonium acetate. It is convenient to use about 2 litres of solvent e.g. acetic acid per mole of anisil. The reaction is conveniently effected at the reflux temperature of the reaction mixture, but lower temperatures will also lead to the desired product. The precise proportion between the reactants, the concentration of each reactant and the reaction temperature is not of vital importance.

b) the reduction of a compound of the formula:—

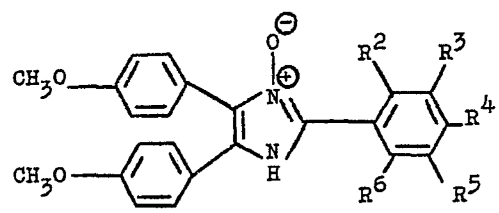

III

where $R^2$, $R^3$, $R^4$, $R^5$ and $R^6$ are as hereinbefore defined, whereby a compound of formula I as hereinbefore defined is obtained.

The reduction is advantageously effected in the presence of zinc, preferably in powder form, and is conveniently effected at the reflux temperature of the reaction mixture.

The compound of formula III is preferably first prepared by reacting anisil monoxime with ammonium acetate and a compound of formula II as hereinbefore defined.

The reaction and reduction may, for example, be effected in a manner analogous to the method of H. Lettau: Z. Chem. *11*, 10 (1971). Thus anisil monoxime, ammonium acetate and the appropriate aldehyde are reacted together advantageously in the presence of a lower alkanoic acid, e.g. acetic acid, and the 3-oxide thus obtained is reduced, preferably *in situ* i.e. without isolation, conveniently by the addition of zinc preferably in the form of a powder. It is convenient to use the anisil monoxime and aldehyde in equimolar quantities in which case it is advantageous to use about five times the equimolar amount of ammonium acetate. The amount of solvent used is conveniently 2 litres per mole of anisil monoxime. The reaction is preferably effected at the reflux temperature of the reaction mixture.

According to a still further feature of the present invention there are provided pharmaceutical or veterinary compositions for the treatment of human patients or domestic mammals comprising as active ingredient at least one compound of formula I as hereinbefore defined in association with a pharmaceutical or veterinary carrier or excipient.

The compositions may be presented in the form suitable for oral, topical, rectal or parenteral administration or in a form for inhalation. Thus, for example, the compositions may be solid or liquid and may take the form of granules, tablets, coated tablets, capsules, syrups, suppositories, ointments, creams, emulsions, suspensions, drops or injectable solutions, such compositions comprising carriers or excipients conventionally used in the pharmaceutical and veterinary art.

Advantageously, the compositions may be formulated as dosage units, each unit being adapted to supply a fixed dose of active ingredient. Tablets, coated tablets and capsules are examples of suitable dosage unit forms. Each dosage unit preferably contains 10 to 500 mg of active ingredient, the total daily dosage is 2—5 mg/kg body weight.

The following Examples illustrate the preparation of compounds according to the invention:—

Example 1

2-(p-Fluorophenyl)-4,5-bis(p-methoxyphenyl)imidazole

800 ml of acetic acid, 310 g (4.0 mole) of ammonium acetate, 108 g (0.4 mole) of anisil and 50 g

(0.4 mole) of *p*-fluorobenzaldehyde are refluxed together for one hour. Boiling water is then added to the clear solution until a slight turbidity persists and the heating is stopped. After cooling the white precipitate formed is filtered off, washed with 2N ammonia and then with water, dried and recrystallized from 2-propanol.

Yield of recrystallized product: 119.7 g (80%). The product is homogenous by TLC and shows a melting point of 207.5—210.5°C.

| | | C | H | N |
|---|---|---|---|---|
| $C_{23}H_{19}FN_2O_2$ | Calc. | 74.28 | 5.15 | 7.53 |
| (374.40) | Found | 73.95 | 4.91 | 7.51 |

The mass spectrum shows a molecular ion at $\frac{m}{e}$ = 374 (base peak), $\frac{m}{e}$ = 359 (M—15),

metastable $\frac{m^x}{e}$ = 345 (corresponding to the fragmentation $M^+ \rightarrow (M—15)^+$).

The IR-spectrum shows the following characteristics: 3450 $cm^{-1}$ (broad) NH; 3100—2900 $cm^{-1}$ NH and CH (aromatic); 2840 $cm^{-1}$ CH ($OCH_3$) 1620, 1525, 1505 $cm^{-1}$ aromatic ring system; 1250, 1035 $cm^{-1}$ aryl-$OCH_3$; 835 $cm^{-1}$ 1,4-substituted benzene rings.

The NMR-spectrum shows the following characteristics:

δ(ppm) = 3.87, singlet (6H) ($CH_3O$) × 2

7.0; 7.6, AB quartet (8H) ($C_6H_4OCH_3$) × 2

7.1, triplet (2H) } ($C_6H_4F$)

7,9, doublet of doublets (2H) }

~10.5, broad singlet (1H) (N*H*).

## Example 2

### 2-(2,4-Difluorophenyl)-4,5-bis(p-methoxyphenyl)imidazole

210 ml of acetic acid, 84 g (1.09 mole) of ammonium acetate, 28.4 g (0.105 mole) of anisil and 14.95 g (0.105 mole) of 2,4-difluorobenzaldehyde are refluxed together for one hour. The heating is stopped and boiling water is added to the clear solution until a slight turbidity persists.

After cooling the white precipitate formed is filtered off, wahed with 2N ammonia and then with water, dried and recrystallised from 2-propanol.

Yield of recrystallised product: 34.9 g (85%). The product is homogenous by TLC and shows a melting point of 137—8°C.

| | | C | H | N |
|---|---|---|---|---|
| $C_{23}H_{18}F_2N_2O_2$ | Calc. | 70.38 | 4.62 | 7.18 |
| (392.39) | Found | 70.45 | 4.50 | 7.11 |

The mass spectrum shows a molecular ion at $\frac{m}{e}$ = 392 (base peak), $\frac{m}{e}$ = 377 (M—15),

metastable $\frac{m^x}{e}$ = 363 (corresponding to the fragmentation $M^+ \rightarrow (M—15)^+$).

The IR-spectrum shows the following characteristics: 3470 $cm^{-1}$ (sharp) NH; 3500—3400 $cm^{-1}$ (broad) NH; 3100—3000 $cm^{-1}$ CH (aromatic); 2845 $cm^{-1}$ CH ($OCH_3$); 1620, 1525, 1500 $cm^{-1}$ aromatic ring system; 1250, 1035 $cm^{-1}$ aryl-O—$CH_3$; 855, 845, 835 $cm^{-1}$ 1,4-substituted benzene rings.

The NMR-spectrum shows the following characteristics:

$\delta$(ppm) = 3.82, singlet (6H) ($CH_3O$) × 2

6.9; 7.5, AB quartet (8H) ($C_6H_4OCH_3$) × 2

6,9 multiplets (2H) } ($C_6H_3F_2$)

8.4, multiplet (1H) }

9.5, broad singlet (1H) (N*H*)

2,4-Difluorobenzaldehyde can be prepared from 2,4-difluorotoluene according to Gunther Lock: Montash. *90,* 680 (1959).

Example 3

2-(p-Fluorophenyl)-4,5-bis(p-methoxyphenyl)imidazole

28.5 g (0.1 mole) of anisil monoxime, 20 g (0.26 mole) of ammonium acetate, 12.4 g (0.1 mole) of *p*-fluorobenzaldehyde and 200 ml of acetic acid are refluxed together. After 2 hours, 20 g of zinc powder are added to the reaction mixture in small portions, and the refluxing is continued for a further 4 hours. After cooling, the precipitate of zinc acetate and unreacted zinc powder is filtered off and discarded. The filtrate is added dropwise to one litre of water with vigorous stirring. The precipitate formed is filtered off, washed by suspension first in 2N ammonia and then in water, dried and finally recrystallised from 2-propanol.

Yield of recrystallised product: 29 g (74%)

Melting point: 208—210°C

IR spectrum identical with the IR-spectrum of the product from Example 1.

Example 4

2-(2,4-Difluorophenyl)-4,5-bis(p-methoxyphenyl)imidazole

28.5 g (0.1 mole) of anisil monoxime, 20 g (0.26 mole) of ammonium acetate, 14.2 g (0.1 mole) of 2,4-difluorobenzaldehyde and 200 ml of acetic acid are refluxed together. After 2 hours, 20 g of zinc powder are added to the reaction mixture in small portions, and the refluxing is continued for a further 4 hours. After cooling the precipitate of zinc acetate and unreacted zinc powder is filtered off and discarded. The filtrate is added dropwise to one litre of water under vigorous stirring. The precipitate formed is filtered off, washed by suspension first in 2N ammonia and then in water, dried and finally recrystallised from 2-propanol.

Yield of recrystallised product: 32.3 g (82%).

Melting point: 137—8°C.

IR-spectrum identical with the IR-spectrum of the product from Example 2.

Example 5

2-(m-fluorophenyl)-4,5-bis(p-methoxyphenyl)imidazole

380 ml of acetic acid, 151 g (1.96 mole) of ammonium acetate, 51 g (0.19 mole) of anisil and 23.5 g (0.189 mole) of m-fluorobenzaldehyde are refluxed together for one hour. Boiling water is then added to the clear solution until a slight turbidity persists and the heating is stopped. After cooling the white precipitate formed is filtered off, washed with 2N ammonia and then with water, dried and recrystallised from 2-propanol.

Yield of recrystallised product: 40.5 g (57%).

The product is homogenous by TLC and shows a melting point of 221.7—223.7°C.

The IR-spectrum (KBr) shows the following characteristics: 3440 $cm^{-1}$ (broad) NH; 3100—2900 $cm^{-1}$ NH and CH (aromatic); 2840 $cm^{-1}$ CH ($OCH_3$); 1620, 1525 and 1495 $cm^{-1}$ aromatic ring system; 1250 and 1035 $cm^{-1}$ aryl-$OCH_3$; 835 $cm^{-1}$ 1,4-substituted benzene rings.

The 60 MHz $^1H$ NMR-spectrum of a 10% solution in DMSO-$d_6$ shows the following characteristics:

$\delta$(ppm) = 3.85 singlet (3H) ($CH_3O$)

3.90 singlet (3H) ($CH_3O$)

7.1; 7.7 AB quartet (8H) ($C_6H_4OCH_3$) × 2

~7.3 (pattern not resolved) (1H) } ($C_6H_4F$)

~7.8 (pattern not resolved) (1H) } ($C_6H_4F$)

8.1 multiplet (2H) } ($C_6H_4F$)

13.1 broad singlet (1H) (NH)

Example 6

2-(o-fluorophenyl)-4,5-bis(p-methoxyphenyl)imidazole

380 ml of acetic acid, 151 g (1.96 mole) of ammonium acetate, 51 g (0.19 mole) of anisil and 23.5 g (0.189 mole) of o-fluorobenzaldehyde are refluxed together for one hour. Boiling water is then added to the clear solution until a slight turbidity persists and the heating is stopped. After cooling the white precipitate formed is filtered off, washed with 2N ammonia and then with water, dried and recrystallised from 2-propanol.

Yield of recrystallised product: 46.0 g (65%).

The product is homogenous by TLC and shows a melting point of 97.2—104.4°C.

The IR-spectrum shows the following characteristics: 3460 $cm^{-1}$ (broad) NH; 3100—2900 $cm^{-1}$ NH and CH (aromatic); 2845 $cm^{-1}$ CH ($OCH_3$); 1620, 1525 and 1500 $cm^{-1}$ aromatic ring system; 1250 $cm^{-1}$ and 1035 $cm^{-1}$ and aryl-$OCH_3$; 840 $cm^{-1}$ 1,4-substituted benzene rings.

The 60 MHz $^1H$ NMR-spectrum of a 10% solution in DMSO-$d_6$ shows the following characteristics:

$\delta$(ppm) = 3.80 singlet (3H) ($CH_3O$)

3.85 singlet (3H) ($CH_3O$)

7.1; 7.6 AB quartet (8H) ($C_6H_4OCH_3$) ×2

~7.3 (pattern not resolved) (2H) } ($C_6H_4F$)

~7.5 (pattern not resolved) (1H) } ($C_6H_4F$)

8.2 doublet of doublets (1H) } ($C_6H_4F$)

12.8 broad singlet (1H) (NH)

Example 7

2-(2,5-difluorophenyl)-4,5-bis(p-methoxyphenyl)imidazole

200 ml of acetic acid, 77 g (1.0 mole) of ammonium acetate, 27 g (0.1 mole) of anisil and 14.95 g (0.105 mole) of 2,5-difluorobenzaldehyde are refluxed together for one hour. Boiling water is then added to the clear solution until a slight turbidity persists and the heating is stopped. After cooling the white precipitate formed is filtered off, washed with 2N ammonia and then with water, dried and recrystallised from 2-propanol.

Yield of recrystallised product: 28.2 g (69%).

The product is homogenous by TLC and shows a melting point of 140.1—141.6°C.

The IR-spectrum (KBr) shows the following characteristics: 3460 $cm^{-1}$ (broad) NH; 3100—2900 $cm^{-1}$ NH and CH (aromatic); 2850 $cm^{-1}$ CH ($OCH_3$); 1622, 1525 and 1500 $cm^{-1}$ aromatic ring systems; 1250 $cm^{-1}$ (assym.) and 1035 $cm^{-1}$ (sym.) C—O—C; 838 $cm^{-1}$ 1,4-disubstituted benzene rings.

The 60 MHz $^1$H NMR-spectrum of a 10% solution in DMSO-$d_6$ shows the following characteristics:

δ(ppm) = 3.90 singlet (6H) ($CH_3O$) × 2

7.3; 7.9 AB quartet (8H) ($C_6H_4OCH_3$) × 2

7.6 multiplet (2H) } ($C_6H_3F_2$)

8.2 multiplet (1H) }

13.1 broad singlet (1H) (NH)

The 2,5-difluorobenzaldehyde used as a starting compound can be prepared from 1,4-difluorobenzene in the same way as described for 2,6-difluorobenzaldehyde by A. M. Roe et al.: J.Med. Chem. *11* (1968) 814. $b_{15}$ = 54—56°C.

Example 8

2-(2,6-difluorophenyl)-4,5-bis(p-methoxyphenyl)imidazole

200 ml of acetic acid, 77 g (1.0 mole) of ammonium acetate, 27 g (0.1 mole) of anisil and 14.95 g (0.105 mole) of 2,6-difluorobenzaldehyde are refluxed together for one hour. Boiling water is then added to the clear solution until a slight turbidity persists and the heating is stopped. After cooling the white precipitate formed is filtered off, washed with 2N ammonia and then with water, dried and recrystallised from 2-propanol/diisopropylether (1/1).

Yield of recrystallised product: 16.7 g (42%).

The product is homogenous by TLC and shows a melting point of 156.4—157.4°C.

The IR-spectrum (KBr) shows the following characteristics: 3460 $cm^{-1}$ (broad) NH; 3140—2900 $cm^{-1}$ CH (aromatic); 2845 $cm^{-1}$ CH ($OCH_3$); 1620, 1525 and 1500 $cm^{-1}$ aromatic ring systems; 1250 $cm^{-1}$ (assym.) and 1035 $cm^{-1}$ (sym.) C—O—C; 850 and 840 $cm^{-1}$ 1,4-disubstituted benzene rings.

The 60 MHz $^1$H NMR-spectrum of a 10% solution in DMSO-$d_6$ shows the following characteristics:

δ(ppm) = 3.83 singlet (3H) ($CH_3O$)

3.80 singlet (3H) ($CH_3O$)

7.0; 7.5 AB quartet (4H) ($C_6H_4OCH_3$)

7.1; 7.6 AB quartet (4H) ($C_6H_4OCH_3$)

~7.2 hidden single (1H) ($C_6H_3F_2$)

7.45 triplet (2H) ($C_6H_3F_2$)

12.9 ppm broad singlet (1H) (NH)

The 2,6-difluorobenzaldehyde used as a starting compound can be prepared as described by A. M. Roe et al.: J.Med. Chem. *11* (1968) 814. $b_{21}$ = 88—90°C.

Example 9

2-(2,3-difluorophenyl)-4,5-bis(p-methoxyphenyl)imidazole

200 ml of acetic acid, 77 g (1.0 mole) of ammonium acetate, 27 g (0.1 mole) of anisil and 14.95 g (0.105 mole) of 2,3-difluorobenzaldehyde are refluxed together for one hour. Boiling water is then added to the clear solution until a slight turbidity persists and the heating is stopped. After cooling the white precipitate formed is filtered off, washed with 2N ammonia and then with water, dried and recrystallised from 2-propanol.

Yield of recrystallised product: 29.3 g (75%).

The product is homogenous by TLC and shows a melting point of 155.4—158.5°C.

The IR-spectrum (KBr) shows the following characteristics: 3460 $cm^{-1}$ (broad) NH; 3140—2900 $cm^{-1}$ CH (aromatic); 2850 $cm^{-1}$ CH ($OCH_3$); 1625, 1525 and 1495 $cm^{-1}$ aromatic ring systems; 1240 $cm^{-1}$ (assym.) and 1040 $cm^{-1}$ (sym.) C—O—C; 840 $cm^{-1}$ 1,4-disubstituted benzene rings.

The 60 MHz $^1H$ NMR-spectrum of a 10% solution in DMSO-$d_6$ shows the following characteristics;

$\delta$(ppm) = 3.80 singlet (6H) ($CH_3O$) × 2

7.0; 7.5 AB quartet (8H) ($C_6H_4OCH_3$) × 2

~7.3 multiplet (2H) } ($C_6H_3F_2$)

7.8 multiplet (1H) }

12.6 broad singlet (1H) (NH)

The 2,3-difluorobenzaldehyde used as a starting compound can be prepared from 1,2-difluorobenzene in the same way as described for 2,6-difluorobenzaldehyde by A. M. Roe et al.: J.Med. Chem. *11* (1968) 814. $b_{15}$ = 60—61°C.

Example 10

2-(3,4-difluorophenyl)-4,5-bis(p-methoxyphenyl)imidazole

200 ml of acetic acid, 77 g (1.0 mole) of ammonium acetate, 27 g (0.1 mole) of anisil and 14.95 g (0.105 mole) of 3,4-difluorobenzaldehyde are refluxed together for one hour. Boiling water is then added to the clear solution until a slight turbidity persists and the heating is stopped. After cooling the white precipitate formed is filtered off, washed with 2N ammonia and then with water, dried and recrystallised from 2-propanol.

Yield of recrystallised product: 29.7 g (76%).

The product is homogenous by TLC and shows a melting point of 188—191.1°C.

The IR-spectrum (KBr) shows the following characteristics: 3450cm$^{-1}$ (broad) NH; 3100—2900 cm$^{-1}$ CH (aromatic); 2850 cm$^{-1}$ CH ($OCH_3$); 1620, 1530 and 1510 cm$^{-1}$ aromatic ring systems; 1255 cm$^{-1}$ (assym.) and 1040 cm$^{-1}$1 (sym.) C—O—C; 840 cm$^{-1}$ 1,4-disubstituted benzene rings.

The 60 MHz $^1H$ NMR-spectrum of a 10% solution in DMSO-$d_6$ shows the following characteristics:

$\delta$(ppm) = 3.93 singlet (6H) ($CH_3O$) × 2

7.7; 7.1 AB quartet (8H) ($C_6H_4OCH_3$) × 2

7.3 multiplet (2H) } ($C_6H_3F_2$)

7.9—8.7 multiplet (1H) }

13.1 broad singlet (1H) (NH)

The 3,4-difluorobenzaldehyde used as a starting compound can be prepared as follows: 1,2-difluorobenzene is brominated by a method analogous to the one described in Organic Synthesis Coll. Vol. I p. 123. From the resulting 3,4-difluorobromobenzene, 3,4-difluorophenylmagnesiumbromide is prepared in diethylether, and this Grignard reagent is added dropwise to a solution of N-methylformanilide in tetrahydrofuran. From the reaction mixture, 3,4-difluorobenzaldehyde is obtained. $b_{17}$ = 80—82°C.

Example 11

2-(3,5-difluorophenyl)-4,5-bis(p-methoxyphenyl)imidazole

200 ml of acetic acid, 77 g (1.0 mole) of ammonium acetate, 27 g (0.1 mole) of anisil and 14.95 g (0.105 mole) of 3,5-difluorobenzaldehyde are refluxed together for one hour. Boiling water is then added to the clear solution until a slight turbidity persists and the heating is stopped. After cooling the white precipitate formed is filtered off, washed with 2N ammonia and then with water, dried and recrystallised from 2-propanol.

Yield of recrystallised product: 22 g (56%).

The product is homogenous by TLC and shows a melting point of 234—237°C.

The IR-spectrum (KBr) shows the following characteristics: 3450 cm$^{-1}$ (broad) NH; 3140—2900 cm$^{-1}$ CH (aromatic); 2850 cm$^{-1}$ CH ($OCH_3$); 1635, 1530, 1500 and 1460 cm$^{-1}$ aromatic ring systems; 1255 cm$^{-1}$ (assym.) and 1040 cm$^{-1}$ (sym.) C—O—C; 840 cm$^{-1}$ 1,4-disubstituted benzene rings.

The 60 MHz $^1H$ NMR-spectrum of a 10% solution in DMSO-$d_6$ shows the following characteristics:

$\delta$(ppm) = 3.85 singlet (6H) ($CH_3O$) × 2

7.1; 7.6 AB quartet (8H) ($C_6H_4OCH_3$) × 2

7.3 multiplet (1H) } ($C_6H_3F_2$)

7.9 multiplet (2H) }

~13 broad singlet (1H) (NH)

The 3,5-difluorobenzaldehyde used as a starting compound can be prepared as follows: 3,5-difluorophenylmagnesiumbromide can be prepared as described by A. Roe and W. F. Little: J.Org.Chem. *20* (1955) 1577. A solution of 3,5-difluorophenylmagnesiumbromide in diethylether is added dropwise to a solution of N-methylformanilide in tetrahydrofuran. By working up this reaction mixture, 3,5-difluorobenzaldehyde is obtained. $b_{55}$ = 86—88°C.

## Claims

1. Imidazole derivative of the formula:—

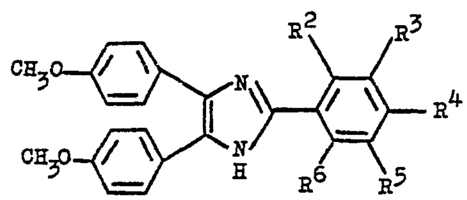

I

wherein one or two of the substituents $R^2$, $R^3$, $R^4$, $R^5$ and $R^6$ represent a fluorine atom while the remaining substituents are hydrogen atoms.

2. Imidazole derivative as per claim 1, viz. 2-(2-fluorophenyl)-4,5-bis(p-methoxyphenyl)-imidazole.

3. Imidazole derivative as per claim 1, viz. 2-(3-fluorophenyl)-4,5-bis(p-methoxyphenyl)-imidazole.

4. Imidazole derivative as per claim 1, viz. 2-(4-fluorophenyl)-4,5-bis(p-methoxyphenyl)-imidazole.

5. Imidazole derivative as per claim 1, viz. 2-(2,3-difluorophenyl)-4,5-bis(p-methoxyphenyl)-imidazole.

6. Imidazole derivative as per claim 1, viz. 2-(2,4-difluorophenyl)-4,5-bis(p-methoxyphenyl)-imidazole.

7. Imidazole derivative as per claim 1, viz. 2-(2,5-difluorophenyl)-4,5-bis(p-methoxyphenyl)-imidazole.

8. Imidazole derivative as per claim 1, viz. 2-(2,6-difluorophenyl)-4,5-bis(p-methoxyphenyl)-imidazole.

9. Imidazole derivative as per claim 1, viz. 2-(3,4-difluorophenyl)-4,5-bis(p-methoxyphenyl)-imidazole.

10. Imidazole derivative as per claim 1, viz. 2-(3,5-difluorophenyl)-4,5-bis(p-methoxyphenyl)-imidazole.

11. Process for the preparation of an imidazole derivative as per claim 1 consisting
1) of a reaction of anisil with a compound of the formula:—

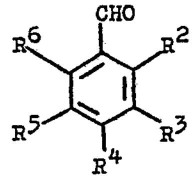

II

where one or two of the substituents $R^2$, $R^3$, $R^4$, $R^5$ and $R^6$ represent a fluorine atom, while the remaining substituents represent hydrogen atoms, and ammonium acetate, or

2) of a reduction of a compound of the formula:—

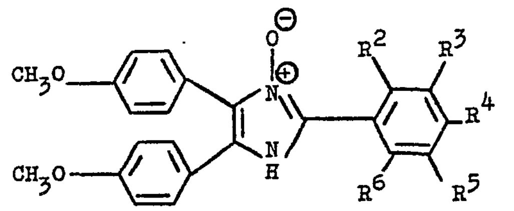

III

12. Process according to claim 11, where the reaction of anisil with a compound of formula II and ammonium acetate is performed in the presence of a protic solvent.

13. Process according to claim 11, where in reacting anisil with a compound of formula II and ammonium acetate, a quantity of five times the equimolar amount of ammonium acetate is used.

14. Process according to claim 12, where approximately two litres of solvent per mole of anisil is used.

15. Process according to claim 12, where the reaction is performed at the reflux temperature of the reaction mixture.

16. Process according to claim 11, wherein the reaction of the compound of formula III is performed in the presence of zinc.

17. Process according to claim 16, wherein zinc powder is used.

18. Process according to claim 11, wherein a compound of formula III is prepared by reaction of anisil monoxime with ammonium acetate and an appropriate aldehyde in the presence of a lower alkanoic acid and the reduction is performed without isolation of the resulting compound.

19. A pharmaceutical or veterinary preparation containing as an active ingredient at least a compound of formula I

**Patentansprüche**

1. Imidazolderivate der allgemeinen Formel

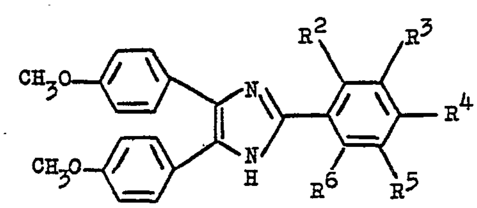

(I)

worin ein oder zwei der Substituenten $R^2$, $R^3$, $R^4$, $R^5$ und $R^6$ für ein Fluoratom stehen und die restlichen Substituenten Wasserstoffatome bedeuten.

2. Ein Imidazolderivat nach Anspruch 1, nämlich 2-(2-Fluorphenyl)-4,5-bis(p-methoxyphenyl)-imidazol.

3. Ein Imidazolderivat nach Anspruch 1, nämlich 2-(3-Fluorphenyl)-4,5-bis(p-methoxyphenyl)-imidazol.

4. Ein Imidazolderivat nach Anspruch 1, nämlich 2-(4-Fluorphenyl)-4,5-bis(p-methoxyphenyl)-imidazol.

5. Ein Imidazolderivat nach Anspruch 1, nämlich 2-(2,3-Difluorphenyl)-4,5-bis(p-methoxyphenyl)-imidazol.

6. Ein Imidazolderivat nach Anspruch 1, nämlich 2-(2,4-Difluorphenyl)-4,5-bis(p-methoxyphenyl)-imidazol.

7. Ein Imidazolderivat nach Anspruch 1, nämlich 2-(2,5-Difluorphenyl)-4,5-bis(p-methoxyphenyl)-imidazol.

8. Ein Imidazolderivat nach Anspruch 1, nämlich 2-(2,6-Difluorphenyl)4,5-bis(p-methoxyphenyl)-imidazol.

9. Ein Imidazolderivat nach Anspruch 1, nämlich 2-(3,4-Difluorphenyl)-4,5-bis(p-methoxyphenyl)-imidazol.

10. Ein Imidazolderivat nach Anspruch 1, nämlich 2-(3,5-Difluorphenyl)-4,5-bis(p-methoxyphenyl)imidazol.

11. Verfahren zur Herstellung eines Imidazolderivates nach Anspruch 1 dadurch gekennzeichnet, dass man

1) Anisil mit einer Verbindung der Formel

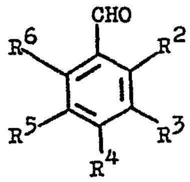

(II)

worin ein oder zwei Substituenten $R^2$, $R^3$, $R^4$, $R^5$ und $R^6$ ein Fluoratom bedeuten und die restlichen Substituenten für Wasserstoffatome stehen, sowie mit Ammoniumacetat umsetzt, oder

2) eine Verbindung der Formel

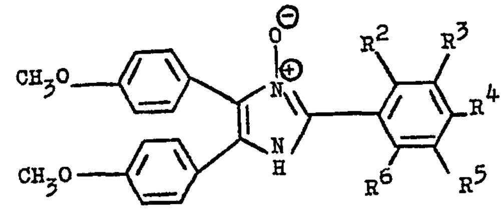

(III)

reduziert.

12. Verfahren nach Anspruch 11, dadurch gekennzeichnet, dass man die Reaktion von Anisil mit einer Verbindung der Formel (II) und Ammoniumacetat in Gegenwart eines protischen Lösungsmittels ausführt.

13. Verfahren nach Anspruch 11, dadurch gekennzeichnet, dass man bei der Reaktion von Anisil mit einer Verbindung der Formel (II) und Ammoniumacetat das 5-fache der äquimolaren Menge von Ammoniumacetat einsetzt.

14. Verfahren nach Anspruch 12, dadurch gekennzeichnet, dass man ungefähr 2 l Lösungsmittel je Mol Anisil anwendet.

15. Verfahren nach Anspruch 12, dadurch gekennzeichnet, dass man die Umsetzung bei der Rückflusstemperatur des Reaktionsgemisches ausführt.

16. Verfahren nach Anspruch 11, dadurch gekennzeichnet, dass man die Reduktion der Verbindung der Formel (III) in Gegenwart von Zink ausführt.

17. Verfahren nach Anspruch 16, dadurch gekennzeichnet, dass man Zinkpulver einsetzt.

18. Verfahren nach Anspruch 11, dadurch gekennzeichnet, dass man die Verbindung der Formel (III) durch Reaktion von Anisilmonoxim mit Ammoniumacetat und einem entsprechenden Aldehyd in Gegenwart einer niederen Alkancarbonsäure herstellt und die Reduktion ohne Isolierung der erhaltenen Verbindung ausführt.

19. Eine pharmazeutische oder veterinärmedizinische Zubereitung, enthaltend als Wirkstoff mindestens eine Verbindung der Formel (I).

**Revendications**

1. Dérivé d'imidazole de la formule:

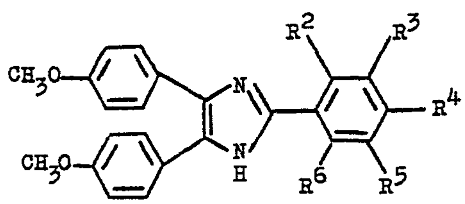

I

dans laquelle un ou deux des substituants $R^2$, $R^3$, $R^4$, $R^5$ et $R^6$ représente un atome de fluor, tandis que les substituants restants sont des atomes d'hydrogène.

2. Dérivé d'imidazole selon la revendication 1, à savoir le 2-(2-fluorophényl)-4,5-bis-(p-méthoxyphényl)-imidazole.

3. Dérivé d'imidazole selon la revendication 1, à savoir le 2-(3-fluorophényl)-4,5-bis(p-méthoxyphényl)imidazole.

4. Dérivé d'imidazole selon la revendication 1, à savoir le 2-(4-fluorophenyl)-4,5-bis(p-méthoxyphényl)imidazole.

5. Dérivé d'imidazole selon la revendication 1, à savoir le 2-(2,3-difluorophényl)-4,5-bis(p-méthoxyphényl)imidazole.

6. Dérivé d'imidazole selon la revendication 1, à savoir le 2-(2,4-difluorophényl)-4,5-bis(p-méthoxyphényl)imidazole.

7. Dérivé d'imidazole selon la revendication 1, à savoir le 2-(2,5-difluorophényl)-4,5-bis(p-méthoxyphényl)imidazole.

8. Dérivé d'imidazole selon la revendication 1, à savoir le 2-(2,6-difluorophényl)-4,5-bis(p-méthoxyphényl)imidazole.

9. Dérivé d'imidazole selon la revendication 1, à savoir le 2-(3,4-difluorophényl)-4,5-bis(p-méthoxyphényl)imidazole.

10. Dérivé d'imidazole selon la revendication 1, à savoir le 2-(3,5-difluorophényl)-4,5-bis(p-méthoxyphényl)imidazole.

11. Procédé de préparation d'un dérivé d'imidazole selon la revendication 1, consistant:

1) à faire réagir l'anisile avec un composé de la formule:

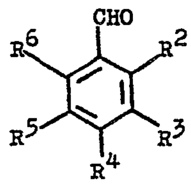

II

dans laquelle un ou deux des substituants $R^2$, $R^3$, $R^4$, $R^5$ et $R^6$ représentent un atome de fluor tandis que les substituants restants représentent des atomes d'hydrogène, et l'acétate d'ammonium, ou

2) à réduire un composé de la formule:

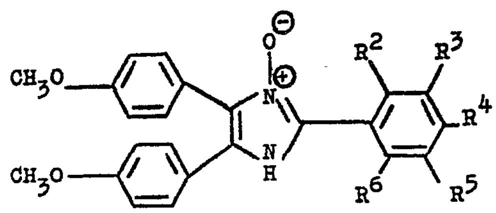

III

12. Procédé selon la revendication 11, dans lequel on conduit la réaction de l'anisile avec un composé de formule II et de l'acétate d'ammonium en présence d'un solvant protique.

13. Procédé selon la revendication 11, dans lequel, en faisant réagir l'anisile avec un composé de formule II et de l'acétate d'ammonium, on utilise une quantité d'acétate d'ammonium égale à cinq fois la quantité équimolaire.

14. Procédé selon la revendication 12, dans lequel on utilise environ 2 litres de solvant par mole d'anisile.

15. Procédé selon la revendication 12, dans lequel on conduit la réaction à la température de reflux du mélange réactionnel.

16. Procédé selon la revendication 11, dans lequel on conduit la réduction du composé de formule III en présence de zinc.

17. Procédé selon la revendication 16, dans lequel on utilise de la poudre de zinc.

18. Procédé selon la revendication 11, dans lequel on prépare un composé de formule III en faisant réagir la monoxime d'anisile avec de l'acétate d'ammonium et un aldéhyde approprié en présence d'un acid alcanoïque inférieur et on conduit la réduction sans isoler le composé obtenu.

19. Préparation pharmaceutique ou vétérinaire contenant comme ingrédient actif au moins un composé de formule I.